# FASCICULE DE BREVET EUROPEEN

(11) **EP 1 971 270 B1**
(45) Date de publication et mention de la délivrance du brevet: **22.08.2012**
(21) Numéro de dépôt: 06831064.8
(22) Date de dépôt: 06.11.2006
(51) Int. Cl.: A61B 17/00, A61B 17/08, A61N 5/06

(54) **SYSTEME POUR LE TRAITEMENT DE PLAIES DE LA PEAU, PANSEMENT ET EQUIPEMENT D 'ACTIVATION BIOCHIMIQUE POUR LA MISE EN OEUVRE D'UN TEL SYSTEME**
SYSTEM ZUR BEHANDLUNG VON HAUTWUNDEN, VERBAND UND BIOCHEMISCHES AKTIVIERUNGSGERÄT ZUR VERWENDUNG MIT DIESEM SYSTEM
SYSTEM FOR TREATMENT OF SKIN WOUNDS, DRESSING, AND BIOCHEMICAL ACTIVATION EQUIPMENT FOR THE USE OF SUCH A SYSTEM

(30) Priorité: 09.01.2006 FR 0600160
(43) Date de publication de la demande: 24.09.2008
(73) Titulaire: EKKYO, 13590 Meyreuil (FR)
(72) Inventeur: CORNIL, Alain, F-13090 Aix-en-Provence (FR)
(74) Mandataire: Barbot, Willy
(86) Numéro de dépôt international: PCT/FR2006/002462
(87) Numéro de publication internationale: WO 2007/080239

(56) Documents cités:
- WO-A-97/17025
- FR-A- 2 598 088
- US-A- 5 156 613

## Description

La présente invention concerne le domaine de la réparation de plaies de la peau.

On connaît, dans l'état de la technique, différentes solutions consistant à améliorer la suture et la cicatrisation par apport d'une énergie externe. Les lèvres de la plaie sont rapprochées et maintenues par un pansement, qui peut éventuellement comporter des principes actifs activables pour la source d'énergie externe.

La demande de brevet international W09717025 décrit un procédé de traitement consistant à la fixation d'un matériau matriciel renfermant un composant protéinique non collagène sur un tissu. On place d'abord ce matériau matriciel sur un emplacement cible du tissu, puis on applique une énergie sur le matériau matriciel. Le composant protéinique non collagène est d'un type tel que, lorsque de l'énergie est appliquée en une quantité appropriée, il se produit une fixation de la matrice au tissu.

La demande de brevet européen EP265470 décrit un dispositif pour l'assemblage des lèvres d'une plaie comportant un laser dont la longueur d'onde d'émission est choisie de manière à pouvoir réaliser une soudure tissulaire et assembler les lèvres d'une plaie et une pièce de maintien apte à être assujettie au tissu autour de la plaie de manière à maintenir affrontées les lèvres de cette dernière, au moins pendant l'exposition de la plaie audit rayonnement laser. La pièce de maintien comporte au moins une région apte à être positionnée au-dessus de la plaie et suffisamment transparente à la longueur d'onde du rayonnement laser pour que l'énergie de ce dernier, après traversée de ladite région, soit suffisante pour réaliser la soudure tissulaire recherchée.

Document US-A-5156613 discloses a device according to the preamble of claim 1.

Les équipements d'activation tel qu'une source laser ne sont pas sans danger, et leur manipulation peut provoquer des accidents lorsque le faisceau est dirigé par inadvertance vers l'oeil d'une personne présente près de la zone d'intervention.

Le but est d'éviter ces inconvénients des solutions de l'art antérieur.

À cet effet, l'invention concerne un système selon la revendication 1. pour le traitement de plaies de la peau.

De préférence, la distance de détection du moyen d'identification est inférieure à cinquante centimètres.

La source d'énergie est constituée par une source laser.

Il est décrit que le moyen d'identification peut être constitué par au moins un aimant permanent et en ce que le capteur de l'équipement peut être un capteur magnétique associé à un calculateur pour calculer une distance en fonction des signaux électromagnétiques détectés.

Il est également décrit que le moyen d'identification peut être constitué par des marquages optiques et en ce que le capteur de l'équipement peut être un capteur d'image associé à un calculateur pour calculer une distance en fonction de l'image détectée.

Il est decrit que le moyen d'identification peut être constitué par un transpondeur.

Avantageusement, le moyen d'identification comporte un identifiant unique du modèle de pansement associé.

De préférence, les paramètres de fonctionnement de la source d'énergie sont commandés en fonction dudit identifiant unique.

L'invention concerne également un pansement selon la revendication 5.

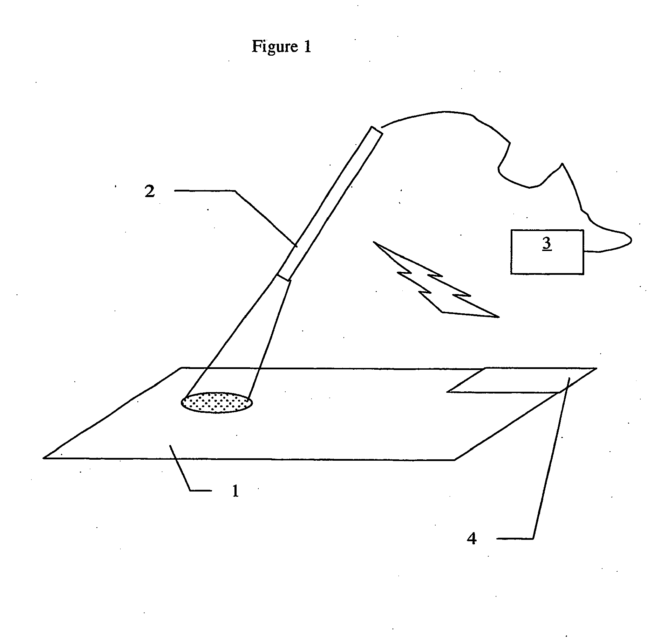

L'invention sera mieux comprisse à la lecture de la description qui suit, se référant à la figure annexée correspondant à une vue schématique d'un équipement selon l'invention.

Le pansement (1) est formé par un film transparent décrit dans la demande de brevet européen EP265470. Il coopère avec une source laser (2) commandé par un boîtier de contrôle (3) assurant l'alimentation et le pilotage de la source laser.

Le pansement (1) présente une étiquette radiofréquence (4). Cette étiquette comprend de façon connue une boucle d'induction assurant l'alimentation d'un circuit comprenant une mémoire dans laquelle est enregistré un identifiant du type de pansement.

Ces informations permettent d'optimiser les paramètres de la source d'énergie associée, notamment la puissance, la durée et la fréquence des impulsions.

La pièce à main (2) comprenant le laser est munie d'une alimentation permettant d'activer l'étiquette (4) par un champ électromagnétique capté par la boucle d'induction. Elle comprend également un capteur destiné à recevoir les signaux électromagnétiques émis par l'étiquette radiofréquence (4) lorsque celle-ci est alimentée.

L'activation du laser est conditionnée par la détection d'un signal d'identification d'une étiquette. En l'absence d'un tel signal, le laser est en position de veille, et évite ainsi tout risque d'accident, même lorsqu'il est orienté par inadvertance en direction d'une personne.

En particulier, lorsque la pièce à main (2) est éloignée du pansement d'une distance supérieure à la portée de l'étiquette radiofréquence (4), le laser est inactif.

Les différents paramètres de commande du laser en fonction des différents types de pansements sont enregistrés dans la mémoire du bloc de commande du laser, par exemple sous forme d'une table. Ces paramètres peuvent être actualisés, notamment en cas de commercialisation d'un nouveau type de pansement, par une liaison avec un équipement informatique externe, ou encore par une saisie à l'aide d'une interface de saisie incorporée au boîtier de contrôle (3).

L'invention n'est pas limitée à une interaction entre une étiquette radiofréquence et un capteur.

L'interaction peut être réalisée selon une variante équivalente par des marqueurs magnétiques apposés sur le pansement. Ces marqueurs sont constitués par des éléments d'aimants minces, ou par des particules magnétiques aimantées. La pièce à main comporte dans ce cas un ou plusieurs capteurs magnétosensibles, par exemple des sondes de Hall, délivrant un signal fonction de l'amplitude du champ détecté dans une ou plusieurs directions. Ces signaux sont exploités par un calculateur pour déterminer la distance et la direction des marqueurs magnétiques apposés sur le pansement, par une méthode connue de triangulation.

L'interaction peut être réalisée selon une autre variante équivalente par l'apposition d'un marquage optique, par exemple fluorescent, excité par une source secondaire montée dans la pièce à main. La pièce à main comprend alors un capteur optique, par exemple un capteur CCD (capteur à transfert de charge) associé à un calculateur procédant à une analyse de l'image détectée pour calculer une distance, et éventuellement une orientation de la pièce à main par rapport au marquage du pansement. Ce marquage peut prendre la forme d'un code matriciel, ou de figures géométriques permettant de déduire la distance en fonction de la taille et la déformation de l'image détecté par le capteur de la pièce à main.

Le pansement peut être constitué par un film transparent simple, assurant le rapprochement et le maintien temporaire des lèvres de la plaie et permettant le transfert de l'énergie fournie par la pièce à main.

Il peut également comporter des revêtements actifs participant aux réactions biochimiques sous l'effet de l'excitation par une source d'énergie.

La source d'énergie décrite est un faisceau laser.

## Revendications

1. Système pour le traitement de plaies de la peau comprenant une source laser (2) pour l'activation d'un effet biochimique de cicatrisation et au moins un pansement destiné à être apposé sur la plaie avant la réalisation de l'étape d'activation par ladite source laser **caractérisé en ce que** :
- ledit pansement (1) comporte une étiquette radiofréquence (4) interagissant sans contact avec un capteur,
- laquelle étiquette radiofréquence peut être activée au moyen d'un champ électromagnétique généré par une alimentation et capté par une boucle d'induction de ladite étiquette radiofréquence,
- laquelle boucle d'induction assurant l'alimentation d'un circuit comprenant une mémoire dans laquelle est enregistré un identifiant du type de pansement, ces informations permettant d'optimiser la puissance, la durée et la fréquence des impulsions de la source laser associée,
- lequel capteur commande le fonctionnement de la source laser seulement lorsque la distance entre le capteur et ladite étiquette radiofréquence est inférieure à une valeur seuil et optimise en outre la puissance, la durée et la fréquence des impulsions de la source laser en fonction des signaux électromagnétiques émis par l'étiquette radiofréquence lorsque celle-ci est alimentée.

2. Système de traitement de plaies selon la revendication 1, **caractérisé en ce que** la source laser est commandée par un calculateur recevant un signal provenant du capteur apte à interagir avec l'étiquette radiofréquence.

3. Système de traitement de plaies selon l'une au moins des revendications précédentes, **caractérisé en ce que** l'étiquette radiofréquence comporte un identifiant unique du modèle de pansement associé.

4. Système de traitement de plaies la revendication 3, **caractérisé en ce que** les paramètres de fonctionnement de la source laser sont commandés en fonction dudit identifiant unique.

5. Pansement pour la mise en oeuvre d'un système selon l'une quelconque des revendications précédentes, **caractérisé en ce qu'**il comporte une étiquette radiofréquence.

6. Equipement d'activation biochimique pour la mise en oeuvre d'un système selon l'une au moins des revendications 1 à 4, **caractérisé en ce qu'**il comporte une source laser commandée par un calculateur (3) recevant un signal provenant d'un capteur apte à interagir avec l'étiquette radiofréquence du pansement.

## Claims

1. A skin wound treatment system comprising a laser source (2) for activating a biochemical wound-healing effect controlled and at least one dressing designed to be affixed to the wound before the activation stage is performed using said laser source, **characterized in that**:
- said dressing (1) includes a radio frequency identifcation tag (4) that interacts without contact with a sensor,
- said radio frequency identification tag (4) can be activated by means of an electromagnetic field generated by a power supply and detected by an induction loop from said radio frequency identification tag,
- said induction loop providing power to a circuit that includes a memory in which a dressing type identifier is recorded, said information being used to control the power, the duration and the frequency of the pulses of the laser source,
- said sensor enables operation of the laser source upon the distance between the sensor and the identification means being less than a determined threshold value and further optimizes the power, the duration and the frequency of the pulses of the laser source depending on electromagnetic signals emitted by RFID tag when the latter is supplied with power.

2. A skin wound treatment system as claimed in claim 1, **characterized in that** the laser source is controlled by a calculator that receives a signal from a sensor suitable for interacting with the RFID tag.

3. A skin wound treatment system as claimed in any one of the previous claims, **characterized in that** the RFID tag includes a unique identifier for the dressing model associated with it.

4. A skin wound treatment system as claimed in claim 3, **characterized in that** the operating settings of the laser source are controlled according to said unique identifier.

5. A dressing for use in a system as claimed in any one of the previous claims, **characterized in that** it comprised a RFID tag.

6. A biochemical activation device for use in a system as claimed in any of claims I to 4, **characterized in that** it includes a laser source controlled by a calculator (3) that receives a signal from a sensor suitable for interacting with the RFID tag incorporated in a dressing.

## Patentansprüche

1. System zur Behandlung von Wunden auf der Haut mit einer Laserquelle (2) zur Aktivierung eines biochemischen Wundheilungsprozesses und mindestens einem Pflaster, das vor der Aktivierungsphase durch die oben genannte Laserquelle auf die Wunde aufzubringen ist, das sich dadurch auszeichnet, dass
- das oben genannte Pflaster (1) einen RFID-Transponder (4) enthält, welcher kontaktlos mit einem Sensor interagiert,
- dieser RFID-Transponder durch ein von einer Quelle gespeistes und einer Induktionsschleife des oben genannten RFID-Transponders empfangenes elektromagnetisches Feld aktiviert werden kann,
- diese Induktionsschleife einen Kreislauf mit einem Speicher versorgt, in welchem ein Identifikator enthalten ist vom Typ des Pflasters, wobei mit diesen Informationen die Leistung, Dauer und Frequenz der Signale der verbundenen Laserquelle optimiert werden können,
- deren Sensor die Laserquelle nur dann steuert, wenn der Abstand zwischen Sensor und RFID-Transponder unter einem bestimmten Schwellenwert liegt und darüber hinaus die Leistung, Dauer und Frequenz der Signale der Laserquelle entsprechend den elektromagnetischen Signale optimiert, die vom RFID- Transponder ausgesendet werden, wenn dieser versorgt wird.

2. System zur Wundenbehandlung gemäß dem Anspruch 1, das sich dadurch auszeichnet, dass die Laserquelle von einem Rechner gesteuert wird, der ein Signal aus einem Sensor empfängt, das mit dem RFID-Transponder interagieren kann.

3. System zur Wundenbehandlung gemäß mindestens einem der obigen Ansprüche, das sich dadurch auszeichnet, dass der RFID-Transponder einen einzigartigen Identifikator des damit verbundenen Pflastermodells enthält.

4. System zur Wundenbehandlung gemäß dem Anspruch 3, das sich dadurch auszeichnet, dass die Funktionsparameter der Laserquelle entsprechend dem oben genannten einzigartigen Identifikator gesteuert werden.

5. Pflaster zur Verwendung eines Systems gemäß einer der oben aufgeführten Ansprüche, welches sich dadurch auszeichnet, dass es einen RFID-Transponder enthält.

6. Vorrichtung zur biochemischen Aktivierung zur Verwendung eines Systems gemäß mindestens einem der obigen Ansprüche 1 bis 4, die sich dadurch auszeichnet, dass sie eine Laserquelle enthält, welche von einem Rechner (3) gesteuert wird, der ein Signal aus einem Sensor empfängt, das mit dem RFID-Transponder des Pflasters interagieren kann.
